# EUROPÄISCHE PATENTANMELDUNG

(11) **EP 3 649 971 A1**
(43) Veröffentlichungstag der Anmeldung: **13.05.2020**
(21) Anmeldenummer: 18205704.2
(22) Anmeldetag: 12.11.2018
(51) Int. Cl.: A61B 17/32, A61B 17/34, A61B 17/00, A61B 90/00

(54) **CHIRURGISCHE VORRICHTUNG ZUM ZERKLEINERN UND ABTRANSPORTIEREN VON KÖRPERGEWEBE**

(71) Anmelder: Kreuz, Gerold, 02997 Wittichenau (DE)
(72) Erfinder: Kreuz, Gerold, 02997 Wittichenau (DE)
(74) Vertreter: Schaad, Balass, Menzl & Partner AG

(57) **Zusammenfassung**

Eine chirurgische Vorrichtung (10) zum Zerkleinern und Abtransportieren von Körpergewebe. Die Vorrichtung (10) umfassend ein Führungselement (20), welches einen Durchgangskanal (30) und eine mit dem Durchgangskanal (30) strömungsverbundene Abführöffnung (40) zur Abführung vom zerkleinerten Körpergewebe aufweist, wobei der sich distal zur Abführöffnung erstreckender Bereich des Führungselements einen intraabdominalen Abschnitt (70) aufweist, und ein in den Durchgangskanal einführbares, schaftförmiges Zerkleinerungsinstrument (142), welches in seinem distalen Endbereich (164) einen eine Förderschnecke (166) aufweisenden Zerkleinerungs- und Förderabschnitt (170) umfasst, wobei bei vollständig eingeführtem Zerkleinerungsinstrument (142) der Zerkleinerungs- und Förderabschnitt (170) über das distale Ende (50) des Führungselements (20) vorsteht, und dass ein Schirm (210) an einem distalen Endbereich (50) des intraabdominalen Abschnitts (70) befestigt ist, wobei der Schirm (210) in seiner Ruheposition zusammengefaltet parallel zur Längsachse des Führungselements (20) und in seiner Aufspannposition nach distal in radialer Richtung von der Längsachse (L) des Führungselements (20) weg verläuft, um ein trichterförmiges Haltemittel für das zu zerkleinernde Körpergewebe zu bilden.

## Beschreibung

Die Erfindung betrifft eine chirurgische Vorrichtung zum Zerkleinern und Abtransportieren von Körpergewebe gemäss dem Oberbegriff des Anspruchs 1.

Mittels minimalinvasiver Verfahren können Organteile bzw. ganze Organe in einer Körperhöhle eines Patienten, insbesondere in der Bauchhöhle, operiert und gegebenenfalls aus dieser entfernt werden. In letztem Fall müssen Organteile bzw. Organe durch relativ kleine Öffnungen, üblicherweise 0,3 bis 2 cm langen Hautschnitt, extrahiert werden. Bekannte chirurgische Vorrichtungen zum Zerkleinern und Abtransportieren von Körpergewebe können im Rahmen von solchen minimalinvasiven chirurgischen Verfahren verwendet werden, um Organteile bzw. ganze Organe aus der Körperhöhle herauszuziehen. Nach entsprechenden Leitlinien in der Medizin muss das zu entfernende Körpergewebe in einem Schutzbeutel aufgefangen und unter Schutz dieses Schutzbeutels entfernt werden, um die Streuung von Geweberesten, z. B. auch Tumorzellen, in der Körperhöhle zu vermeiden. Dabei besteht die Gefahr, dass beim Zerkleinern des zu entfernenden Körpergewebes der Schutzbeutel und das umliegende, intakte Gewebe verletzt werden.

Aus US 5 569 284 A ist eine intraabdominale Vorrichtung bekannt, welche eine Handhabevorrichtung und einen an die Handhabevorrichtung anschliessenden intraabdominalen Abschnitt umfasst. Der intraabdominale Abschnitt beinhaltet ein längliches rohrförmiges Element, das über eine drehbare Kupplung an der Handhabevorrichtung befestigt ist. Das rohrförmige Element weist ein geschlossenes und abgerundetes distales Ende und eine axiale Bohrung auf, in welcher eine schaftförmige, von einem Aktuator angetriebene Förderschnecke zum Zerkleinern und Abtransportieren von Körpergewebe drehbar angeordnet ist. Im distalen Endbereich des röhrenförmigen Elements sind eine seitliche Öffnung und eine schwenkbar zwischen einer geöffneten Position und einer geschlossenen Position bewegliche Abdeckklappe angeordnet, welche Abdeckklappe dazu bestimmt ist, die Öffnung selektiv zu bedecken und freizulegen, um den Zugang des Körpergewebes zur Förderschnecke zu ermöglichen. In der geöffneten Position bietet die Abdeckklappe Zugang für das Körpergewebe, um in die Öffnung zur Förderschnecke zu gelangen. Wird die Abdeckklappe von der geöffneten in die geschlossene Position bewegt, übt sie eine Kraft aus, um das Körpergewebe gegen die Förderschnecke zur Zerkleinerung zu drücken. In der geschlossenen Position verhindert die Abdeckklappe, dass die Förderschnecke unbeabsichtigtes Material zur Zerkleinerung kontaktiert. Eine Verletzung des Schutzbeutels während des Zerkleinerungsvorgangs ist dadurch vermieden, dass einerseits das distale Ende des rohrförmigen Elements geschlossen und abgerundet ausgebildet ist, und anderseits die Förderschnecke beim Zerkleinern und Abtransportieren des Körpergewebes in der axialen Bohrung des rohrförmigen Elements untergebracht ist. Da das zu zerkleinernde Gewebe lediglich durch die seitliche Öffnung im distalen Endbereich mit der Förderschnecke in Kontakt kommt, können allerdings jeweils nur kleine Körpergewebeteile zerkleinert werden. Dies hat zur Folge, dass der Zerkleinerungsvorgang und die Operation entsprechend lang dauern.

Aus DE 10 2016 011 185 A1 ist ein Zerkleinerungsvorrichtung für die Chirurgie bekannt, umfassend einen wenigstens teilweise konisch zulaufenden, rohrförmigen Schaft, welcher einen distalen zylindrischen Endbereich aufweist. In einer Ausführungsform ist ein Zerkleinerungsorgan im Innern der distalen Hälfte des Endbereichs angeordnet, welches über einen Aktuator betätigt wird. Um das zu zerkleinernde Körpergewebe dem Zerkleinerungsorgan zuzuführen, weist das Zerkleinerungsvorrichtung eine Zuführvorrichtung auf, welche als medizinischer Fangkorb ausgebildet ist. Über eine definierte Anzahl von in entsprechenden Führungshülsen verlaufenden Korbdrähten wird der Fangkorb ausgebildet, in welchen das zu zerkleinernde Gewebe beispielsweise über eine Greif- oder Klemmvorrichtung eingelegt werden kann, welche über einen weiteren Zugang in die Körperhöhle eingeführt sein kann. Der Fangkorb ist dabei beweglich ausgebildet, d. h. eine Betätigung des Fangkorbs kann die Korbdrähte von einer geschlossenen Position in eine zur Aufnahme von Gewebe angepasste, offene Position bewegen. Weiterhin kann der Fangkorb in Richtung des Zerkleinerungsorgans bewegt werden, wodurch ein Zerkleinern des Körpergewebes mittels des Zerkleinerungsorgans bewirkt wird. Durch die Anordnung des Zerkleinerungsorgans im zylindrischen Endbereich des Schafts wirkt der Schaft als Schutzhülle. Das Risiko von einer Verletzung des Schutzbeutels oder des umliegenden Gewebes wird durch diese schützende Wirkung deutlich herabgesetzt. Da der Zerkleinerungsvorgang zusätzliche Schritte erfordert, nämlich das Einlegen des zu zerkleinernden Körpergewebes in den Fangkorb und das Bewegen des Fangkorbs in Richtung des Zerkleinerungsorgans zur Zerkleinerung des sich im Fangkorb befindenden Körpergewebes, hat dies zur Folge, dass der Zerkleinerungsvorgang und die Operation entsprechend lang dauern.

Aufgabe der vorliegenden Erfindung ist es somit, eine chirurgische Vorrichtung zum Zerkleinern und Abtransportieren von Körpergewebe zur Verfügung zu stellen, welche einen sicheren und schnellen Zerkleinerungsvorgang ermöglicht. Hierzu ist mit dem Begriff "sicher" gemeint, dass beim Zerkleinern des zu entfernenden Körpergewebes, welches in einem Schutzbeutel enthalten sein kann, das Risiko, gegebenenfalls den Schutzbeutel und/oder das umliegende, intakte Gewebe zu verletzen vermieden wird.

Diese Aufgabe wird erfindungsgemäss durch eine chirurgische Vorrichtung zum Zerkleinern und Abtransportieren von Körpergewebe gemäss Anspruch 1 gelöst. Bevorzugte Ausführungsformen der Erfindung sind in den abhängigen Ansprüchen wiedergegeben.

Die Erfindung betrifft eine chirurgische Vorrichtung zum Zerkleinern und Abtransportieren von Körpergewebe, umfassend ein, eine Längsachse der Vorrichtung definierendes Führungselement. Das Führungselement ist vorzugsweise wenigstens annähernd rotationssymmetrisch zur Längsachse ausgebildet und weist einen, sich wenigstens annähernd rotationssymmetrisch entlang der Längsachse erstreckenden Durchgangskanal und eine mit dem Durchgangskanal strömungsverbundene Abführöffnung zur Abführung vom zerkleinerten Körpergewebe. Der Durchgangskanal bildet somit einen Kanal, der sich durchgehend vom distalen Ende bis zum proximalen Ende des Führungselements erstreckt.

Dabei bezeichnet der Begriff "distal" die vom Benutzer der erfindungsgemässen Vorrichtung abgewandte, ferner liegende Seite; dementsprechend bezeichnet der Begriff "proximal" die dem Benutzer zugewandte, näherliegende Seite.

Ferner weist das Führungselement einen sich distal zur Abführöffnung vorzugsweise bis zu dessen distalen freien Ende erstreckenden, den Durchgangskanal umfassenden, wenigstens annähernd zylindrischen intraabdominalen Abschnitt auf, welcher dazu bestimmt ist, mit dem distalen Ende voraus in eine Körperhöhle, insbesondere über einen Hautschnitt, eingeführt zu werden. Die zylindrische äussere Form des intraabdominalen Abschnitts ermöglicht eine einfache Einführung des intraabdominalen Abschnitts in die Körperhöhle. Ferner stellt die zylindrische innere Form des intraabdominalen Abschnitts eine zuverlässige Führung eines in das Führungselement eingeführten Zerkleinerungsinstruments sicher.

Das Führungselement umfasst ebenfalls einen an das proximale Ende des intraabdominalen Abschnitts anschliessenden, vorzugsweise zur Längsachse rotationssymmetrischen Abführabschnitt, welcher den Durchgangskanal umschliesst und an welchen die Abführöffnung angeordnet ist. Der Innendurchmesser des Durchgangskanals im Abführabschnitt ist mindestens gleich gross, vorzugsweise grösser als der Innendurchmesser des Durchgangskanals im intraabdominalen Abschnitt.

In einer bevorzugten Ausführungsform kann der Innendurchmesser des Durchgangskanals ab dem distalen Ende des Abführabschnitts, das an den intraabdominalen Abschnitt anschliesst, nach proximal zunehmen, vorzugsweise kontinuierlich zunehmen bis zu einem maximalen Innendurchmesser, und somit einen sich nach proximal erweiternden, wenigstens annähernd konischen, ersten Bereich umfassen. Anschliessend an den ersten Bereich kann der Innendurchmesser des Durchgangskanals gleich dem maximalen Innendurchmesser über einen zweiten Bereich konstant bleiben. In einem an den zweiten Bereich anschliessenden dritten Bereich kann der Innendurchmesser des Durchgangskanals nach proximal abnehmen, vorzugsweise bis zu dem Innendurchmesser des Durchgangskanals im intraabdominalen Abschnitt abnehmen. Der dritte Bereich bildet folglich einen sich nach proximal verjüngend erstreckenden Abschnitt des Abführabschnitts, der sich bis zum proximalen Ende des Abführabschnitts erstecken kann. Der Abführabschnitt bildet eine Kammer zur Zwischenlagerung vom zerkleinerten Körpergewebe, an die die Abführöffnung angeordnet ist. Vorzugsweise schliesst die Kammer direkt an den intraabdominalen Abschnitt. Diese Anordnung ermöglicht eine Sammlung des zerkleinerten Körpergewebes, welches somit effizient abgesaugt und abgeführt werden kann.

In einer besonders bevorzugten Ausführungsform ist die Abführöffnung durch einen, am Abführabschnitt mit dem Durchgangskanal strömungsverbunden angeordneten, rohrförmigen Anschluss gebildet, wobei das zerkleinerte und abtransportierte Körpergewebe mittels eines an den Anschluss anschliessbaren Saugschlauchs angesaugt und abgeführt wird. Vorzugsweise verläuft der Anschluss radial zur Längsachse. Diese Anordnung ermöglicht eine kontinuierliche Abführung des zerkleinerten Körpergewebes zur Entsorgung, sodass der Abführabschnitt platzsparend ausgebildet sein kann und den Zugriff auf den Hautschnitt oder im Bereich des Hautschnitts nicht einschränkt.

In einer bevorzugten Ausführungsform kann die Abführöffnung durch eine axiale Bohrung im Abführabschnitt ausgebildet sein, wobei ein mit der Bohrung strömungsverbundener Behälter zerkleinertes und abtransportiertes Körpergewebe sammelt, das mittels eines Saugschlauchs angesaugt und abgeführt wird.

In einer besonders bevorzugten Ausführungsform ist das Führungselement zweiteilig ausgebildet und weist einen distalen und einen proximalen Gehäusebereich auf, wobei der distale und der proximale Gehäusebereich luftdicht zusammen verschlossen sind, beispielsweise über eine Gewindeverbindung. Die zweiteilige Ausführung des Führungselements erlaubt eine einfache Reinigung und Kontrolle des Zustandes des Führungselements. Die Trennung zwischen den beiden Gehäusebereichen ist vorzugsweise im Bereich des Abführabschnitts angeordnet, welcher den grössten Durchmesser aufweist. Somit ist die Reinigung besonders einfach.

Ferner schliesst ein zur Längsachse rotationssymmetrischer Dichtungsabschnitt, welcher den Durchgangskanal umschliesst und sich bis zum proximalen Ende des Führungselements erstreckt, an das proximale Ende des Abführabschnitts an. Der Innendurchmesser des Durchgangskanals im Dichtungsabschnitt ist grösser oder gleich dem Innendurchmesser des Durchgangskanals im intraabdominalen Abschnitt. Ferner ist der Innendurchmesser in einem zylindrischen Bereich des Dichtungsabschnitts derart ausgewählt, dass der Dichtungsabschnitt und das eingeführte Zerkleinerungsinstrument den Durchgangskanal im zylindrischen Bereich formgenau wenigstens annähernd vollständig abdichten. Durch diese Anordnung wird sichergestellt, dass ein im Abführabschnitt erzeugter Unterdruck zur Absaugung des zerkleinerten Körpergewebes aufgebaut werden kann und wenigstens annähernd konstant bleibt.

In einer bevorzugten Ausführungsform kann der Innendurchmesser des Durchgangskanals in einem an den zylindrischen Bereich anschliessenden Sitzbereich des Dichtungsabschnitts nach proximal bis zu einem Sitzdurchmesser zunehmen, und anschliessend grösser oder gleich dem Sitzdurchmesser sich vorzugsweise bis zum proximalen Ende des Dichtungsabschnitts erstrecken. Diese Anordnung bildet eine zentrierende Einführhilfe für das Zerkleinerungsinstrument. Ferner bildet der Sitzbereich einen Anschlag, an welchem ein an dem Zerkleinerungsinstrument ausgebildeten Gegenanschlag bei vollständig eingeführtem Zerkleinerungsinstrument anliegt. Somit kann die Bewegung des Zerkleinerungsinstruments nach distal in Längsrichtung gestoppt werden.

Die chirurgische Vorrichtung umfasst ebenfalls das in den Durchgangskanal einführbare, schaftförmige Zerkleinerungsinstrument, welches zumindest in seinem distalen Endbereich einen eine Förderschnecke aufweisenden Zerkleinerungs- und Förderabschnitt zum Zerkleinern und Abtransportieren vom Körpergewebe umfasst.

Der Zerkleinerungs- und Förderabschnitt erstreckt sich vom distalen freien Ende des Zerkleinerungsinstruments und reicht mindestens bis zum proximalen Ende des intraabdominalen Abschnitts, welches an den Abführabschnitt anschliesst, gemessen bei vollständig eingeführten Zerkleinerungs instrument.

Die Förderschnecke erstreckt sich mindestens bis in den intraabdominalen Abschnitt hinein, um das zerkleinerte Körpergewebe nach proximal in den intraabdominalen Abschnitt und weiter in den Abführabschnitt fördern zu können. Vorzugsweise erstreckt sich die Förderschnecke, vom distalen Ende des Führungselements gemessen und bei vollständig eingeführtem Zerkleinerungsinstrument, bis zu einem Drittel des intraabdominalen Abschnitts, um eine effiziente Förderung nach proximal zu gewährleisten. Um die Förderung zu optimieren, kann sich die Förderschnecke weiter nach proximal erstrecken. Es ist auch möglich die Förderschnecke über die ganze Länge des Zerkleinerungs- und Förderabschnitts vorzusehen.

Vorzugsweise ist der Zerkleinerungs- und Förderabschnitt durch einen massiven, zylindrischen Schaftabschnitt gebildet, dessen Schaftdurchmesser kleiner als der Innendurchmesser des Durchgangskanals im intraabdominalen Abschnitt ist.

Die Förderschnecke ist vorzugsweise integral einstückig mit dem Schaftabschnitt ausgebildet und weist eine schraubenförmige Helix auf, deren Aussendurchmesser, d.h. der Aussendurchmesser der Förderschnecke, kleiner als der Innendurchmesser des Durchgangskanals im intraabdominalen Abschnitt ist, um eine gegenseitige Reibung zu vermeiden. Der Spalt zwischen der Förderschnecke und der inneren Wandung des intraabdominalen Abschnitts kann zwischen 0.5 mm bis 2 mm, vorzugsweise 1.0 mm breit sein. Der Aussendurchmesser der Förderschnecke ist so bemessen, dass das in den Durchgangskanal eingeführte Zerkleinerungsinstrument wenigstens annähernd in Längsrichtung geführt wird, um eine zuverlässige Bedienung des Zerkleinerungsinstruments während einer Operation zu erlauben. Auf dieser Weise ist ebenfalls sichergestellt, dass die Förderschnecke das zerkleinerte Körpergewebe optimal nach proximal fordern kann, weil es nicht wesentlich zwischen der Förderschnecke und der inneren Wandung des intraabdominalen Abschnitts nach proximal ausfliessen können.

Die Helix steht in radialer Richtung von dem Schaftabschnitt vor und weist einen rechteckigen Querschnitt auf, dessen radiale Länge grösser als seine axiale Breite ist. Der Gang der Helix ist derart ausgebildet, dass beim Antrieb des Zerkleinerungsinstruments in einer Förderdrehrichtung eine Förderung des zerkleinerten Körpergewebes nach proximal zum Abführabschnitt erfolgt.

In einer bevorzugten Ausführungsform kann die radiale Länge der Helix in einem distalen Endbereich der Förderschnecke kontinuierlich abnehmen, sodass der Aussendurchmesser der Förderschnecke ebenfalls abnimmt. Vorzugsweise ist die radiale Länge der Helix am distalen Ende der Förderschnecke Null, sodass die Helix fluchtend mit dem Schaft kommt. Auf dieser Weise kann das Einbohren des Zerkleinerungsinstrument ins Körpergewebe optimiert werden.

Um die Förderung des zerkleinerten Körpergewebes weiter zu optimieren, ist es auch möglich die Helix zweigängig auszubilden.

Das Zerkleinerungsinstrument ist dazu bestimmt, vom proximalen Ende des Führungselements her in den Durchgangskanal eingeführt zu werden. Im Anwendungsfall durchgreift folglich das Zerkleinerungsinstrument den Durchgangskanal.

Vorzugsweise ist das Zerkleinerungsinstrument einstückig ausgebildet.

Erfindungsgemäss steht, bei vollständig eingeführtem Zerkleinerungsinstrument, der Zerkleinerungs- und Förderabschnitt des Zerkleinerungsinstruments mit einem Teil der Förderschnecke über das distale Ende des Führungselements in axialer Richtung vor, um Körpergewebe zu zerkleinern und abzutransportieren.

Ferner, ist ein Schirm vorhanden, welcher dazu bestimmt ist, an einem distalen Endbereich des intraabdominalen Abschnitts befestigt zu sein. Der Schirm verläuft in seiner Ruheposition zusammengefaltet im Wesentlichen parallel zur Längsachse des Führungselements und in seiner Aufspannposition im Wesentlichen nach distal in radialer Richtung von der Längsachse des Führungselements weg, um ein trichterförmiges Haltemittel für das zu zerkleinernde Körpergewebe zu bilden. Beim Zerkleinern und Abtransportieren des nach üblicher Praxis in einem Schutzbeutel aufgefangen Körpergewebes wird der Schutzbeutel straff um den Schirm und das zu zerkleinernde Körpergewebe herum durch Ziehen des Schutzbeutels nach proximal gehalten, wie dies nachstehend detailliert erläutert wird. Folglich wird der Kontakt des zu zerkleinernden Körpergewebes mit dem Zerkleinerungsinstrument maximiert, sodass die Zerkleinerung effizient verläuft. Entsprechend wird die gesamte Dauer der Operation vorteilhaft reduziert.

Der über das distale Ende des Führungselements in axialer Richtung vorstehende Abschnitt der Förderschnecke bildet einen Bohrabschnitt. Bei vollständig eingeführtem Zerkleinerungsinstrument ist die maximale Länge des Bohrabschnitts erreicht.

In einer bevorzugten Ausführungsform ist der freie Rand der Helix als eine scharfe Schneide im Bohrabschnitt ausgebildet, um Körpergewebe in kleine Stücke effizienter zerkleinern zu können, wenn das Zerkleinerungsinstrument gedreht wird. Vorzugsweise erstreckt sich die Schneide über den gesamten Bohrabschnitt, um möglichst viel Körpergewebe bei der Drehung des Zerkleinerungsinstruments zu zerkleinern, und somit die Dauer der Operation zu reduzieren. Es ist jedoch auch möglich, die Schneide über die ganze Länge der Förderschnecke vorzusehen.

An den Zerkleinerungs- und Förderabschnitt schliesst einen Führungsabschnitt an, dessen Aussendurchmesser kleiner als der Innendurchmesser des Dichtungsabschnitts des Führungselements ist. Der Führungsabschnitt ist mindestens gleich lang wie die gesamte Länge des Abführabschnitts und des Dichtungsabschnitts des Führungselements.

In einer bevorzugten Ausführungsform ist eine vorzugweise zylindrische Dichtungssektion im proximalen Endbereich des Führungsabschnitts ausgebildet, dessen Aussendurchmesser so bemessen ist, dass sie wenigstens annähernd spielfrei in Längsrichtung in den zylindrischen Bereich des Dichtungsabschnitts eingeschoben werden kann. Der Dichtungsabschnitt und die Dichtungssektion sind derart ausgebildet, dass sie sich mindestens teilweise überlappen, wenn das distale Ende des Zerkleinerungs- und Förderabschnitts über das distale Ende des Führungselements in axialer Richtung vorsteht. Somit wird eine wenigstens annähernd vollständig luftdichte Abdichtung des Führungselements proximal zur Abführöffnung gebildet.

In einer bevorzugten Ausführungsform ist der Aussendurchmesser der Förderschnecke, wenigstens annähernd gleich wie der Innendurchmesser des Durchgangskanals in der Dichtungssektion, um die Einführung des Zerkleinerungsinstruments in den Durchgangskanal in Richtung zum intraabdominalen Abschnitt zu unterstützen. Somit wird die Bedienung der Vorrichtung praktischer.

Ferner kann der Führungsabschnitt proximal zur Dichtungssektion eine den Gegenanschlag umfassende Anschlagssektion aufweisen. Der Gegenanschlag kann dadurch gebildet sein, dass der Aussendurchmesser der Anschlagssektion grösser als der Innendurchmesser des zylindrischen Bereichs des Dichtungsabschnitts ist. Diese Anordnung dient dazu, die maximale Erstreckung des Bohrabschnitts während einer Operation festzusetzen. Bei vollständig eingeführtem Zerkleinerungsinstrument liegt der Gegenanschlag an dem Sitzbereich des Führungselements an und ist die Bewegung des Zerkleinerungsinstruments somit nach distal in Längsrichtung gestoppt.

Vorzugsweise ist die Anschlagssektion komplementär zum Sitzbereich ausgebildet, sodass die Anschlagssektion bei vollständig eingeführtem Zerkleinerungsinstrument eine Abdichtung mit dem Sitzbereich bildet, welche ebenfalls dazu beiträgt, die luftdichte Abdichtung des Führungselements proximal zur Abführöffnung zu verbessern.

Das Zerkleinerungsinstrument ist dazu bestimmt, von einem Aktuator rotierend in die Förderdrehrichtung angetrieben zu werden, um Körpergewebe zu zerkleinern und abzutransportieren.

In einer bevorzugten Ausführungsform schliesst ein Kopfabschnitt distal an den Führungsabschnitt des Zerkleinerungsinstruments an, welcher sich wenigstens annähernd rotationssymmetrisch zur Längsachse erstreckt. Der Kopfabschnitt ist dazu bestimmt, mit dem Aktuator drehend antreibbar verbunden zu sein.

Der Kopfabschnitt kann auf seiner Aussenfläche beispielsweise axial verlaufende Nuten aufweisen, welche zur Mitnahme des Kopfabschnitts durch den Aktuator dienen, und formschlüssig in einem Aufnahmehalter des Aktuators gelagert sein.

Der Begriff "Aktuator" bezeichnet eine Vorrichtung, welche einen elektrisch, pneumatisch oder elektromechanisch betriebenen Drehantrieb umfasst, und sind dem Fachmann aus dem Stand der Technik bekannt.

Die chirurgische Vorrichtung kann eine Handhabevorrichtung aufweisen, welche eine Handhabe zum Halten der chirurgischen Vorrichtung und den Aktuator umfasst.

Der Aktuator kann ebenfalls die Bewegung des Zerkleinerungsinstruments in axialer Richtung steuern. Es ist jedoch auch möglich, die axiale Bewegung des Zerkleinerungsinstruments über eine separate, manuelle Bedienung zu steuern, beispielsweise über einen Bedienungsgriff, welcher mit dem Zerkleinerungsinstrument zusammenwirkt.

Zur Herstellung des Führungselements und des Zerkleinerungsinstruments sind Werkstoffe geeignet, welche bereits in der Medizinaltechnik eingesetzt werden und biokompatibel, reinigbar sowie sterilisierbar sind. Vorzugsweise wird der Werkstoff des erfindungsgemässen Führungselements ausgewählt aus der Gruppe von rostfreiem Stahl, Titan, Titanlegierungen mit Aluminium, Vanadium, Tantal, Niob, Nickel, Eisen und Molybdän oder Gemische davon.

In einer bevorzugten Ausführungsform ist der Schirm an dem distalen Endbereich des intraabdominalen Abschnitts auswechselbar befestigbar. Im Anwendungsfall ist der Schirm an dem distalen Endbereich des intraabdominalen Abschnitts befestigt. Da der Schirm auswechselbar befestigt ist, kann er vom intraabdominalen Abschnitt getrennt werden, um eine einfache Reinigung nach der Operation und/oder einen praktischen Austausch des Schirms zu erlauben. Ein auswechselbarer Schirm ist insbesondere für einen Einweggebrauch vorteilhaft.

In einer bevorzugten Ausführungsform ist der Schirm über eine Gewindeverbindung am distalen Endbereich des intraabdominalen Abschnitts lösbar befestigbar. Eine Gewindeverbindung stellt gleichzeitig eine einfache und zuverlässige Verbindung dar, sodass der Schirm sich vom Führungselement während der Operation nicht löst.

In einer besonders bevorzugten Ausführungsform umfasst der intraabdominale Abschnitt ein Aussengewinde und der Schirm ein mit diesem Aussengewinde zusammenwirkendes Innengewinde, sodass der Schirm auf den intraabdominalen Abschnitt aufgeschraubt werden kann. Bei dieser Anordnung findet die Verbindung ausserhalb des Durchgangskanals statt, sodass der freie Innendurchmesser des Durchgangskanals nicht eingeschränkt wird. Dies kann bei einer Operation vorteilhaft sein, wenn dickflüssiges oder stückiges Körpergewebe zerkleinert und abtransportiert werden muss.

In einer weiteren besonders bevorzugten Ausführungsform weist der distale Endbereich des intraabdominalen Abschnitts eine umlaufende Aussparung zur Reduktion der Wanddicke des intraabdominalen Abschnitts und das in der Aussparung ausgebildete Aussengewinde auf. Die Tiefe der Aussparung, in radialer Richtung gemessen, ist derart bemessen, dass die Aussenfläche des montierten Schirms fluchtend mit der Aussenfläche des intraabdominalen Abschnitts kommt. Bei der Befestigung des Schirms bleibt somit der Aussendurchmesser im intraabdominalen Abschnitt unverändert. Folglich wird eine Verdickung der Vorrichtung an der Schnittstelle zwischen dem intraabdominalen Abschnitt und dem Schirm vermieden. Somit wird ein kleinerer Hautschnitt benötigt, um der den Schirm aufweisenden intraabdominalen Abschnitt einführen zu können.

Es ist jedoch ebenfalls möglich, dass der intraabdominale Abschnitt ein Innengewinde und der Schirm ein mit diesem Innengewinde zusammenwirkendes Aussengewinde aufweisen. Bei der Befestigung des Schirms bleibt somit der Aussendurchmesser im intraabdominalen Abschnitt unverändert. Diese Anordnung kann ebenfalls vorteilhaft sein, wenn ein dünnes Führungselement zur Einführung in eine kleine Öffnung während einer Operation benötigt wird.

In einer besonders bevorzugten Ausführungsform ist der Schirm über einen Bajonettverschluss befestigbar, um eine schnelle Befestigung zu ermöglichen.

In einer weiteren bevorzugten Ausführungsform liegt, in axialer Richtung gemessen, das distale Ende des in der maximalen Aufspannposition aufgespannten, montierten Schirms, bei vollständig eingeführtem Zerkleinerungsinstrument, bei dem freien Ende des Zerkleinerungs- und Förderabschnitts. Vorzugsweise steht dieses dem Ende des Zerkleinerungs- und Förderabschnitts gegenüber vor. Dies bedeutet, dass der Bohrabschnitt, auch bei seiner maximalen Erstreckung, in axialer Richtung gemessen, kürzer als der montierte Schirm ist. Somit kann sichergestellt werden, dass sich ausserhalb des Schirmes befindendes Körpergewebe vom Bohrabschnitt nicht berührt wird. Ferner kann ebenfalls sichergestellt werden, dass beim Zerkleinern des in dem Schutzbeutel aufgefangen Körpergewebes der Bohrabschnitt den Schutzbeutel nicht verletzt, selbst wenn der Schutzbeutel straff um den Schirm und das zu zerkleinernde Körpergewebe gehalten wird. Dies erlaubt eine straffere Führung des Schutzbeutels und damit einen effizienteren Abtransport des Körpergewebes.

In einer besonders bevorzugten Ausführungsform ist eine auf den intraabdominalen Abschnitt aufschiebbare Schiebehülse vorgesehen, welche im montierten Zustand auf den intraabdominalen Abschnitt zwischen einer Freigabeposition und einer Klemmposition hin und her schiebbar ist, wobei die Schiebehülse in ihrer Klemmposition den Schirm umgreift und diesen in seiner Ruheposition hält. Die Schiebehülse ist dazu bestimmt, nach proximal entlang des intraabdominalen Abschnitts in ihre Freigabeposition bewegt zu werden, um das Aufspannen des Schirms in seine Aufspannposition freizugeben.

Der Wechsel von der Ruheposition in die Aufspannposition des Schirmes erfolgt durch die Verschiebung der in ihrer Klemmposition auf den Schirm aufgesetzten Schiebehülse in ihre Freigabeposition nach proximal entlang des intraabdominalen Abschnitts. Da der montierte Schirm in Richtung der maximalen Aufspannposition vorgespannt ist, spannt er sich selbsttätig auf. Hingegen erfolgt der Wechsel von der Aufspannposition in die Ruheposition durch die Verschiebung der in ihrer Freigabeposition auf den intraabdominalen Abschnitt aufgesetzte Schiebehülse in ihre Klemmposition nach distal auf den Schirm.

Zur raschen und sicheren Einführung des den montierten Schirm aufweisenden, intraabdominalen Abschnitts in die Körperhöhle ist ein zusammengefalteter Schirm im Hinblick auf die Grösse des Hautschnitts von Vorteil. Ferner ist ein kontrollierter Wechsel von der Ruheposition des Schirmes in seine Aufspannposition nötig, um eine unerwünschte Aufspannung des Schirmes, beispielsweise während der Operation, zu vermeiden. Die verschiebbare Schiebhülse stellt eine besonders konstruktiv einfache und kostengünstige Lösung dar.

Um die Schiebehülse bewegen zu können, kann die Länge der Schiebehülse, in axialer Richtung gesehen, derart bemessen sein, dass sie bei in der Körperhöhle eingeführtem intraabdominalen Abschnitt zumindest teilweise aus der Körperhöhle ragt. Alternativ kann die Schiebehülse von einem durch einen weiteren Hautschnitt eingeführten Instrument verschoben werden.

Der Innendurchmesser der Schiebhülse ist grösser als der Aussendurchmesser des intraabdominalen Abschnitts, sodass die Schiebehülse über den intraabdominalen Abschnitt und gegebenenfalls über den montierten Schirm geschoben werden. Vorzugsweise ist der Innendurchmesser der Schiebhülse so bemessen, dass sie wenigstens annähernd spielfrei in Längsrichtung über den montierten Schirm geschoben werden kann.

Die Schiebhülse kann auf verschiedene Weise montiert werden. Die Schiebhülse kann über den intraabdominalen Abschnitt in die Freigabeposition aufgesteckt werden. Danach kann der Schirm montiert und die Schiebhülse auf den montierten Schirm geschoben werden, um ihn in der Ruheposition zu halten.

Es ist jedoch auch möglich, den zusammengefalteten Schirm mit der auf den Schirm aufgesetzten Schiebehülse vorzubereiten und als Baugruppe zur Verfügung zu stellen. Die Baugruppe kann über den Schirm an den intraabdominalen Abschnitt vor der Einführung des intraabdominalen Abschnitts in die Körperhöhle befestigt werden, wie vorstehend erklärt. Somit kann die chirurgische Vorrichtung in kurzer Zeit vor der Operation vorbereitet werden.

Der aufgespannte Schirm bildet ein trichterförmiges Haltemittel für das zu zerkleinernde Körpergewebe und kann in Umfangsrichtung durchgehend ausgebildet sein. Der Schirm kann jedoch Schirmsegmente aufweisen, welche jeweils durch einen Spalt getrennt sind.

In einer besonders bevorzugten Ausführungsform weist der Schirm einen wenigstens annähernd kreiszylinderförmigen Grundkörper zur Befestigung an dem intraabdominalen Abschnitt und an dem Grundkörper fest angeordnete Schirmsegmente auf. Die Schirmsegmente verlaufen in der Ruheposition im Wesentlichen parallel zur Längsachse des Führungselements und stehen unter einer Vorspannung in Richtung Aufspannposition, um bei Freigabe sich selbsttätig in die Aufspannposition zu bewegen. In der Aufspannposition verlaufen die Schirmsegmente im Wesentlichen nach distal in radialer Richtung von der Längsachse des Führungselements weg. Die Gestaltung des Schirmes mit einem Grundkörper und Schirmsegmenten stellt eine einfache Konstruktion dar, welche günstig hergestellt werden kann. Dies ist insbesondere bei Einwegschirmen vorteilhaft.

Die Schirmsegmente können durchgehend miteinander verbunden sein und eine durchgehende Schirmfläche bilden. Eine solche Ausführungsform kann bei dünnflüssigem Körpergewebe eingesetzt werden, um eine Verteilung des Körpergewebes ausserhalb des Schirmes zu vermeiden.

In einer besonders bevorzugten Ausführungsform weist der Schirm den wenigstens annähernd kreiszylinderförmigen Grundkörper zur Befestigung an dem intraabdominalen Abschnitt und an dem Grundkörper schwenkbar zwischen der Ruheposition und der Aufspannposition gelagerte Schirmsegmente auf. Ferner ist ein mit den Schirmsegmenten über ein Verbindungselement verbundenes Stellglied vorhanden, wobei der Wechsel zwischen der Ruheposition und der Aufspannposition durch die Betätigung des Stellglieds erfolgt. Die Verwendung eines Stellglieds hat den Vorteil, dass die Betätigung ausserhalb der Körperhöhle erfolgt, sodass kein weiterer Hautschnitt benötigt wird, um die Schirmsegmente zu erreichen und deren Position zu ändern.

Das Stellglied kann beispielsweise an der Handhabevorrichtung angeordnet sein. Gegebenenfalls kann der Aktuator ebenfalls die Bewegung des Stellglieds steuern, um den Wechsel zwischen der Ruheposition und der Aufspannposition des Schirmes mit der Steuerung des Zerkleinerungsinstruments zu koordinieren. Somit kann die Bedienung der chirurgischen Vorrichtung vereinfacht werden.

In einer bevorzugten Ausführungsform kann das Stellglied über die Schiebhülse mit den Schirmsegmenten verbunden sein, wobei die Schiebhülse eine Verbindung zu den jeweiligen Schirmsegmenten aufweist.

In einer bevorzugten Ausführungsform ohne Schiebhülse kann das Stellglied über vorzugsweise metallische Drähte, welche vorzugsweise entlang des intraabdominalen Abschnitts geführt sind, mit den jeweiligen Schirmsegmenten verbunden sein.

In einer bevorzugten Ausführungsform können die Schirmsegmente durch zur Längsachse konvexe, trapezförmige und nach distal sich verjüngende Platten ausgebildet sind, welche vom Grundkörper abstehen. Diese einfache Konstruktion ist robust und kann folglich den Druck gut widerstehen, welcher im Anwendungsfall über den Beutel auf das sich unter dem Schirm befindende, zu zerkleinerte Körpergewebe und auf die Schirmsegmente ausgeübt wird.

In einer bevorzugten Ausführungsform sind die Schirmsegmente durch vom Grundkörper abstehende Speichen und an deren freien Enden anschliessende, zungenförmige Platten gebildet. Über die Biegungseigenschaften der Speichen kann die Form des Schirms, insbesondere die Wölbung des Schirms, im Anwendungsfall besonders einfach gestaltet werden, sodass der Schirm an die Form des zu zerkleinerten Körpergewebes flexible angepasst werden kann. Ferner stellen die Platten sicher, dass das zu zerkleinerten Körpergewebe unter dem Schirm gehalten bleiben, um eine effiziente Zerkleinerung zu erlauben.

In einer bevorzugten Ausführungsform weist der Schirm 3 bis 12, bevorzugt 4 bis 6, besonders bevorzugt 4 Schirmsegmente auf. Über die Anzahl der Schirmsegmente kann die Fähigkeit des Schirmes das zu zerkleinerte Körpergewebe zu halten bestimmt werden. Bei dünnflüssigem Körpergewebe kann einen Schirm mit 8 bis 12 Schirmsegmente und bei festem Körpergewebe kann einen Schirm mit 3 bis 6 Schirmsegmente gewählt werden. Mit 4 Schirmsegmenten wird gleichzeitig einen einfachen und effizienten Schirm gebildet.

In einer bevorzugten Ausführungsform sind die Schirmsegmente in Umfangsrichtung am Grundkörper gleichmässig verteilt und gleich ausgebildet.

In einer bevorzugten Ausführungsform weisen die Schirmsegmente ein abgerundetes distales Ende auf, um im Anwendungsfall zu vermeiden, den Schutzbeutel und das umliegende, intakte Gewebe zu verletzen.

Bevorzugt sind die Schirmsegmente gleich lang. Es ist jedoch möglich, dass mindestens einzelne der Schirmsegmente eine unterschiedliche Länge aufweisen, wobei die Mindestlänge der Schirmsegmente länger als die maximale Erstreckung des Bohrabschnitts bei vollständig eingeführtem Zerkleinerungsinstrument ist. Diese Anordnung ermöglicht einen Schirm zu bilden, welcher an die Form des zu zerkleinerten Körpergewebes angepasst ist. Dies kann beispielsweise von Vorteil sein, wenn das zu zerkleinerten Körpergewebe sich länger in eine erste Richtung als in eine andere Querrichtung erstreckt.

Über die Anzahl, die Anordnung und die Dimensionierung des Schirmsegmente kann die Dimensionierung des Schirmes grundsätzlich den verschiedensten Operationsgebieten angepasst werden.

In einem weiteren Aspekt der Erfindung ist ein Einwegschirm für die erfindungsgemässe chirurgische Vorrichtung wie oben beschrieben vorgesehen, wobei der Einwegschirm austauchbar ist. Die Verwendung eines austauchbaren Einwegschirms bietet eine günstige Lösung, um die Kosten beim Einsatz der erfindungsgemässen chirurgischen Vorrichtung zu reduzieren. Wenn der Schirm während der Operation beschädigt wird, kann er ausgetauscht und kann die chirurgische Vorrichtung wiederverwendet werden.

Zur Herstellung des Schirms und der Schiebehülse sind Werkstoffe geeignet, welche bereits in der Medizinaltechnik eingesetzt werden und biokompatibel, reinigbar sowie sterilisierbar sind. Für die Herstellung des in Richtung der Aufspannposition vorgespannten Schirmes sind biokompatible Kunststoffe besonders gut geeignet, weil die Vorspannung des Schirmes bei seiner Herstellung einfach erzeugt werden kann. Kunststoffe sind ebenfalls für Einwegschirme und Einwegschiebehülsen wegen der tiefen Herstellkosten besonders gut geeignet. Vorzugsweise ist ein Einwegschirm aus einem Kunststoff gefertigt, der ausgewählt aus ist der Gruppe bestehend aus Polyethylen (PE), Polypropylen (PP), Polystyrol (PS), Acrylnitrilbutadienstyrol (ABS), Styrolacrylnitril (SAN), Polyamide (PA), vorzugsweise Polyamid 6 (PA 6), Polyamid 6,6 (PA 66), Polyamid 11 (PA 11), Polyamid 12 (PA 12) und Polyamid 61 (PA610), Polycarbonat (PC), Polyvinylchlorid (PVC), Polyethylenterephtalat (PET), Polyetheretherketon (PEEK), Polyacrylnitril (PAN), Polyamidimide (PAI), Polybutylenterephtalat (PBT), thermoplastisches Polyurethan (TPU), Polyester (PES), Polyvinylakohol (PVA), Polymethylmethacrylat (PMMA), Polysulfon PSU, Polyphenylensulfid (PPS), Polyphenylensilfon (PPSU), Polyethersulfon (PES), Polyetherimid (PEI), Perfluoralkoxyalkan (PFA), Polychlortrifluorethylen (PCTFE), Polyvinylidenfluorid (PVDF), Polyphthalimid (PPA), Styrol-Butadien (SB), Acryl-Styrol-Acrylester (ASA), Ethylen-Vinylacetat-Copolymer (EVA), Polyaryletherketon (PAEK)1 Ethylen-Butylacrylat-Copolymer (EBA), Polyoxymethylen (POM), Polybutadien (PBD), Polyisopren (PIP), Polychloropren und/oder Polyalkyvinylether.

Rostfreier Edelstahl oder Titan kann beispielsweise in Ausführungsformen eingesetzt werden, welche an den Grundkörper des Schirmes schwenkbare Schirmsegmente vorsehen und in welchen die Schirmsegmente mechanisch über eine längere Zeit beansprucht werden. Dies kann insbesondere der Fall sein, wenn eine einmalige Verwendung des Schirms bzw. der Schiebehülse nicht vorgesehen ist.

Die chirurgische Vorrichtung wird üblicherweise nach entsprechenden Leitlinien in der Medizin zusätzlich mit dem Schutzbeutel zur Aufnahme von zu zerkleinerndem Körpergewebe verwendet. In diesem Fall wird der Schutzbeutel über den Hautschnitt in die Körperhöhle eingeführt und das zu zerkleinerte Körpergewebe wird mithilfe eines geeigneten Instruments, beispielsweise eines Greifers oder einer Klemme, in den Schutzbeutel hineingelegt werden. Vor dem Zerkleinern des Körpergewebes wird die Öffnung des Schutzbeutels aus der Körperhöhle herausgezogen.

Danach kann der intraabdominale Abschnitt des Führungselements über die Öffnung des Schutzbeutels in die Körperhöhle eingeführt werden. Der intraabdominale Abschnitt weist einen montierten Schirm auf, welcher zusammengefaltet in seiner Ruheposition durch die sich in ihrer Klemmposition befindende, auf den Schirm aufgesetzte Schiebehülse gehalten wird, wie dies für eine bevorzugte, vorstehend beschriebene Ausführform vorgesehen ist.

In einem weiteren Schritt kann die Schiebehülse in ihre Freigabeposition nach proximal entlang des intraabdominalen Abschnitts geschoben werden, um eine Aufspannung des Schirmes zu erlauben. Der aufgespannte Schirm wird derart positioniert, dass das zu zerkleinernde Körpergewebe zwischen dem Schirm und dem Schutzbeutel gehalten wird. Danach wird das Zerkleinerungsinstrument ins Führungselement eingeführt, sodass der Bohrabschnitt mindestens teilweise in das zu zerkleinernde Körpergewebe durchgreift. Bei Betätigung des Aktuators wird das Zerkleinerungsinstrument in Förderdrehrichtung angetrieben und beginnt das Zerkleinerungsinstrument das zu zerkleinernde Körpergewebe zu zerkleinern und in Richtung des Abführabschnitts zur Abführung abzutransportieren.

Während des Betriebs der chirurgischen Vorrichtung wird der Schutzbeutel von der Aussenseite der Körperhöhle durch Ziehen stets straff gegen das zu zerkleinernde Körpergewebe und den Schirm gehalten, um die Zerkleinerung durch das Zerkleinerungsinstrument zu unterstützen. Das distale Ende des in der maximalen Aufspannposition aufgespannten, montierten Schirms, bei vollständig eingeführtem Zerkleinerungsinstrument, steht, in axialer Richtung gemessen, gegenüber dem freien Ende des Zerkleinerungsinstruments, d.h. gegenüber dem freien Ende des Zerkleinerungs- und Förderabschnitts, vorzugsweise vor. Dies bedeutet, dass der Bohrabschnitt, auch bei seiner maximalen Erstreckung, in axialer Richtung gemessen, kürzer als der montierte Schirm ist.

Während des Zerkleinerungsvorgangs wird je nach Fortschritt der Schutzbeutel weiter von der Aussenseite der Körperhöhle gezogen, um ihn straff gegen das zu zerkleinernde Körpergewebe und den Schirm zu halten. Somit kann sichergestellt werden, dass der Zerkleinerungsvorgang so schnell wie möglich durchgeführt und folglich die Dauer der Operation reduziert wird.

Weitere Vorteile und Eigenschaften der Erfindung gehen aus der nachstehenden Beschreibung eines Ausführungsbeispiels hervor, welches anhand der beiliegenden Figuren erläutert wird.

Diese zeigen rein schematisch:
- Fig. 1: das den montierten Schirm aufweisende Führungselement der erfindungsgemässen chirurgischen Vorrichtung in der Vorderansicht und die Schiebehülse im Querschnitt;
- Fig. 2: eine Explosionsansicht der Vorrichtung gemäss Fig. 1;
- Fig. 3: das Zerkleinerungsinstrument in der Vorderansicht;
- Fig. 4: das in Querschnitt dargestellte Führungselement der Fig. 1 zusammen mit dem eingeführten Zerkleinerungsinstrument gemäss Fig. 3;
- Fig. 5: eine weitere Ausführungsform des aufgespannten Schirms in der Vorderansicht;
- Fig. 6: den durch die in Querschnitt dargestellte Schiebehülse zusammengefaltet gehaltenen Schirm gemäss Fig. 5;
- Fig. 7: das in die Körperhöhle eingesetzte, einen aufgespannten, montierten Schirm aufweisende Führungselement gemäss Fig. 1 unter Weglassung der Schiebhülse; und
- Fig. 8: die Vorrichtung gemäss Fig. 7 zusammen mit dem eingeführten Zerkleinerungsinstrument gemäss Fig. 3, dessen maskierte Kontur gestrichelt dargestellt ist.

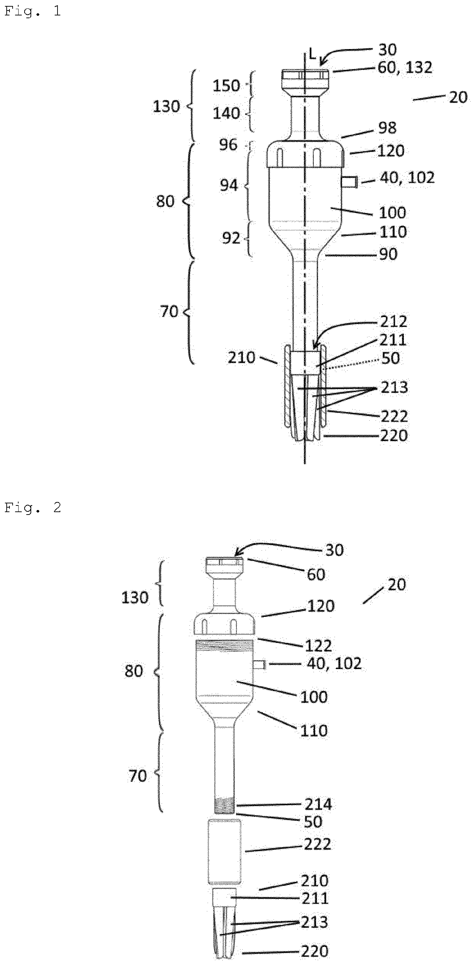

Eine chirurgische Vorrichtung 10 zum Zerkleinern und Abtransportieren von Körpergewebe wird nachstehend bezugnehmend auf die Figurenfolge 1, 2, 3 und 4 beschrieben. Die chirurgische Vorrichtung 10 umfasst ein, eine Längsachse L der Vorrichtung definierendes Führungselement, das auf Fig. 1 und Fig. 2 abgebildet ist. Das Führungselement 20 ist wenigstens annähernd rotationssymmetrisch zur Längsachse L ausgebildet und weist einen, sich wenigstens annähernd rotationssymmetrisch entlang der Längsachse erstreckenden Durchgangskanal 30 und eine mit dem Durchgangskanal strömungsverbundene Abführöffnung 40 zur Abführung vom zerkleinerten Körpergewebe auf. Der Durchgangskanal 30 bildet somit einen Kanal, welcher sich durchgehend vom distalen Ende 50 bis zum proximalen Ende 60 des Führungselements 20 erstreckt.

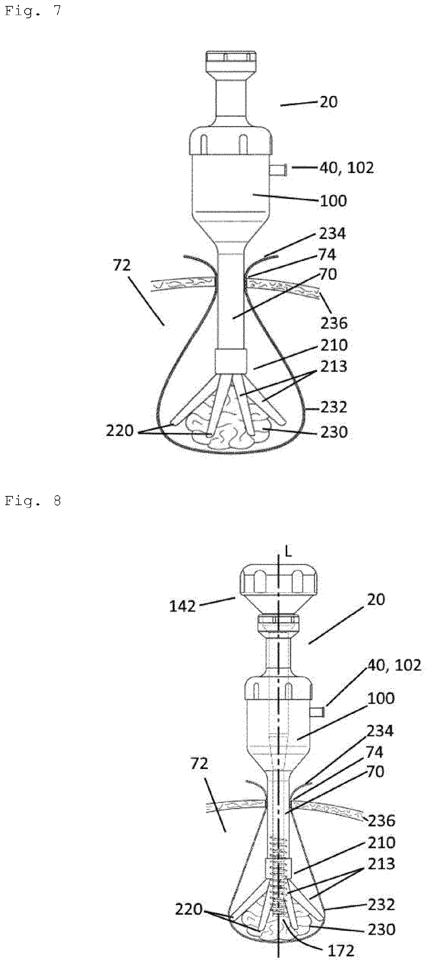

Ferner weist das Führungselement 10 einen sich distal zur Abführöffnung 40 bis zu dessen distalen freien Ende 50 erstreckenden, den Durchgangskanal 30 umfassenden, wenigstens annähernd zylindrischen intraabdominalen Abschnitt 70 auf, welcher dazu bestimmt ist, mit dem distalen Ende 50 voraus in eine Körperhöhle 72, welche in Fig. 7 und 8 ersichtlich ist, über einen Hautschnitt 74, eingeführt zu werden.

Das Führungselement 10 umfasst ebenfalls einen an das proximale Ende 90 des intraabdominalen Abschnitts 70 direkt anschliessenden, zur Längsachse L rotationssymmetrischen Abführabschnitt 80, welcher den Durchgangskanal 30 umschliesst und an welchen die Abführöffnung 40 angeordnet ist. Der Innendurchmesser des Durchgangskanals 30 im Abführabschnitt 80 ist grösser als der Innendurchmesser des Durchgangskanals im intraabdominalen Abschnitt 70.

Der Innendurchmesser des Durchgangskanals 30 nimmt ab dem distalen Ende 90 des Abführabschnitts 30, das an den intraabdominalen Abschnitt 70 direkt anschliesst, nach proximal kontinuierlich bis zu einem maximalen Innendurchmesser zu und bildet somit einen sich nach proximal erweiternden, wenigstens annähernd konischen, ersten Bereich 92. Anschliessend an den ersten Bereich 92 bleibt der Innendurchmesser des Durchgangskanals 30 gleich dem maximalen Innendurchmesser über einen zweiten Bereich 94 konstant. In einem an den zweiten Bereich 94 anschliessenden dritten Bereich 96 nimmt der Innendurchmesser des Durchgangskanals 30 nach proximal bis zu dem Innendurchmesser des Durchgangskanals 30 im intraabdominalen Abschnitt 70 ab. Der dritte Bereich 96 bildet folglich einen sich nach proximal verjüngend erstreckenden Abschnitt des Abführabschnitts 80, welcher sich bis zum proximalen Ende 98 des Abführabschnitts 80 erstreckt. Der Abführabschnitt bildet eine Kammer 100 zur Zwischenlagerung vom zerkleinerten Körpergewebe, an welche die Abführöffnung 40 angeordnet ist.

Die Abführöffnung 40 ist durch einen, am Abführabschnitt mit dem Durchgangskanal 30 strömungsverbunden angeordneten, radial zur Längsachse L verlaufenden, rohrförmigen Anschluss 102 gebildet. Das zerkleinerte und abtransportierte Körpergewebe kann mittels eines an den Anschluss anschliessbaren, nicht dargestellten Saugschlauchs angesaugt und abgeführt werden.

Das Führungselement 20 ist zweiteilig ausgebildet und weist einen distalen 110 und einen proximalen Gehäusebereich 120 auf, wobei der distale und der proximale Gehäusebereich 110 bzw. 120 luftdicht über eine Gewindeverbindung 122 zusammen verschlossen sind. Die Trennung zwischen den beiden Gehäusebereichen 110 bzw. 120 ist im Bereich des Abführabschnitts 80 angeordnet, welcher den grössten Durchmesser aufweist, nämlich der zweite Bereich 94.

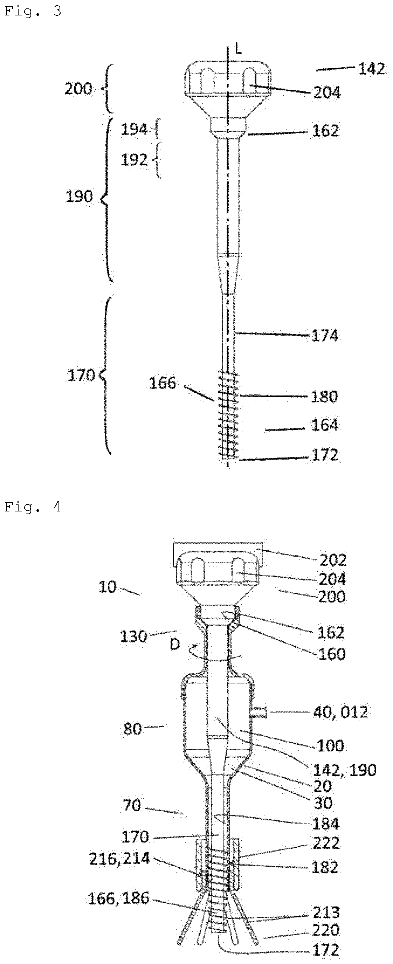

Ferner schliesst ein zur Längsachse L rotationssymmetrischer Dichtungsabschnitt 130, welcher den Durchgangskanal 30 umschliesst und sich bis zum proximalen Ende 60 des Führungselements 20 erstreckt, an das proximale Ende 98 des Abführabschnitts 30 direkt an. Der Innendurchmesser des Durchgangskanals 30 im Dichtungsabschnitt 130 ist grösser als der Innendurchmesser des Durchgangskanals 30 im intraabdominalen Abschnitt 70. Ferner ist der Innendurchmesser in einem zylindrischen Bereich 140 des Dichtungsabschnitts 130 derart ausgewählt, dass der Dichtungsabschnitt 130 und das eingeführte Zerkleinerungsinstrument 142 den Durchgangskanal im zylindrischen Bereich formgenau wenigstens annähernd vollständig abdichten, wie dies auf Fig. 4 ersichtlich ist.

Der Innendurchmesser des Durchgangskanals 30 nimmt in einem an den zylindrischen Bereich 140 direkt anschliessenden Sitzbereich 150 des Dichtungsabschnitts 130 nach proximal bis zu einem Sitzdurchmesser zu. Anschliessend bleibt der Innendurchmesser des Durchgangskanals 30 bis zum proximalen Ende 132 des Dichtungsabschnitts gleich. Diese Anordnung bildet eine zentrierende Einführhilfe für das Zerkleinerungsinstrument 140. Ferner bildet der Sitzbereich einen Anschlag 160, an welchem ein an dem Zerkleinerungsinstrument 140 ausgebildeten Gegenanschlag 162 bei vollständig eingeführtem Zerkleinerungsinstrument 140 anliegt, wie dies ebenfalls auf Fig. 4 ersichtlich ist.

Das in den Durchgangskanal 30 einführbare, einstückig ausgebildete, schaftförmige Zerkleinerungsinstrument 142 umfasst in seinem distalen Endbereich 164 einen eine Förderschnecke 166 aufweisenden Zerkleinerungs- und Förderabschnitt 170 zum Zerkleinern und Abtransportieren vom Körpergewebe.

Der Zerkleinerungs- und Förderabschnitt 170 erstreckt sich vom distalen freien Ende 172 des Zerkleinerungsinstruments 142 und reicht bis zum proximalen Ende 90 des intraabdominalen Abschnitts 70, das an den Abführabschnitt 80 anschliesst, gemessen bei vollständig eingeführten Zerkleinerungsinstrument 142.

Die Förderschnecke 166 erstreckt sich bis in den intraabdominalen Abschnitt 70 hinein, vom distalen Ende 50 des Führungselements 20 gemessen und bei vollständig eingeführtem Zerkleinerungsinstrument 142, bis zu einem Drittel des intraabdominalen Abschnitts 70, um das zerkleinerte Körpergewebe nach proximal in den intraabdominalen Abschnitt 70 und weiter in den Abführabschnitt 80 zu fördern.

Der Zerkleinerungs- und Förderabschnitt 170 ist durch einen massiven, zylindrischen Schaftabschnitt 174 gebildet, dessen Schaftdurchmesser kleiner als der Innendurchmesser des Durchgangskanals 30 im intraabdominalen Abschnitt 70 ist.

Die Förderschnecke 166 ist integral einstückig mit dem Schaftabschnitt 174 ausgebildet und weist eine schraubenförmige Helix 180 auf, deren Aussendurchmesser, d.h. der Aussendurchmesser der Förderschnecke 166, kleiner als der Innendurchmesser des Durchgangskanals 30 im intraabdominalen Abschnitt 70 ist.

Der Spalt 182 zwischen der Förderschnecke 166 und der inneren Wandung 184 des intraabdominalen Abschnitts 70 ist zwischen 0.5 mm bis 2 mm breit.

Die Helix 180 steht in radialer Richtung von dem Schaftabschnitt 174 vor und weist einen rechteckigen Querschnitt auf, dessen radiale Länge grösser als seine axiale Breite ist. Der Gang der Helix 180 ist derart ausgebildet, dass beim Antrieb des Zerkleinerungsinstruments 10 in einer Förderdrehrichtung D eine Förderung des zerkleinerten Körpergewebes nach proximal zum Abführabschnitt 80 erfolgt.

In dieser Ausführungsform bleibt die radiale Länge der Helix 180 konstant über die ganze Förderschnecke 166.

Zum Einbohren des Zerkleinerungsinstrument 142 ins Körpergewebe ist das distale Ende der Helix 180 als eine sich rechtwinklig zur Längsachse L erstreckende Schneide ausgebildet. Ferner ist der freie Rand der Helix 180 ebenfalls als eine Schneide ausgebildet.

Das Zerkleinerungsinstrument 142 ist dazu bestimmt, vom proximalen Ende 60 des Führungselements 20 her in den Durchgangskanal 70 eingeführt zu werden.

Im Anwendungsfall durchgreift folglich das Zerkleinerungsinstrument 142 den Durchgangskanal 70, wie dies auf Fig. 4 ersichtlich ist. Bei vollständig eingeführtem Zerkleinerungsinstrument 142 steht der Zerkleinerungs- und Förderabschnitt 170 des Zerkleinerungsinstruments 142 mit einem Teil 186 der Förderschnecke 166 über das distale Ende des Führungselements 20 in axialer Richtung vor, welcher einen Bohrabschnitt 186 bildet, um Körpergewebe zu zerkleinern und abzutransportieren.

An den Zerkleinerungs- und Förderabschnitt 170 schliesst einen Führungsabschnitt 190 an, dessen Aussendurchmesser kleiner als der Innendurchmesser des Dichtungsabschnitts 130 des Führungselements 20 ist. Der Führungsabschnitt 190 ist gleich lang wie die gesamte Länge des Abführabschnitts 80 und des Dichtungsabschnitts 130 des Führungselements 20.

Eine zylindrische Dichtungssektion 192 ist im proximalen Endbereich des Führungsabschnitts 190 ausgebildet, deren Aussendurchmesser so bemessen ist, dass sie wenigstens annähernd spielfrei in Längsrichtung L in den zylindrischen Bereich 140 des Dichtungsabschnitts 130 eingeschoben werden kann. Der Dichtungsabschnitt 130 und die Dichtungssektion 192 sind derart ausgebildet, dass sie sich mindestens teilweise überlappen, wenn das distale Ende 172 des Zerkleinerungs- und Förderabschnitts 170 über das distale Ende 50 des Führungselements 20 in axialer Richtung vorsteht.

In dieser Ausführungsform ist der Aussendurchmesser der Förderschnecke 166, wenigstens annähernd gleich wie der Innendurchmesser des Durchgangskanals 70 in der Dichtungssektion 192.

Ferner weist der Führungsabschnitt 190 proximal zur Dichtungssektion 192 eine den Gegenanschlag 162 umfassende Anschlagssektion 194 auf. Der Gegenanschlag 162 ist dadurch gebildet, dass der Aussendurchmesser der Anschlagssektion 194 grösser als der Innendurchmesser des zylindrischen Bereichs 140 des Dichtungsabschnitts 130 ist. Bei vollständig eingeführtem Zerkleinerungsinstrument 142 liegt der Gegenanschlag 162 an dem Sitzbereich 150 des Führungselements 20 an und ist die Bewegung des Zerkleinerungsinstruments somit nach distal in Längsrichtung gestoppt. Die Anschlagssektion 162 ist komplementär zum Sitzbereich 150 ausgebildet, sodass die Anschlagssektion 194 bei vollständig eingeführtem Zerkleinerungsinstrument 142 eine Abdichtung mit dem Sitzbereich 150 bildet.

Ein Kopfabschnitt 200 schliesst distal an den Führungsabschnitt 190 des Zerkleinerungsinstruments 142 an, welcher sich wenigstens annähernd rotationssymmetrisch zur Längsachse L erstreckt. Der Kopfabschnitt 200 ist dazu bestimmt, mit einem Aktuator 202, welcher auf Fig. 4 schematisch abgebildet ist, drehend antreibbar verbunden zu sein. Der Kopfabschnitt 200 weist auf seiner Aussenfläche axial verlaufende Nuten 204 auf, welche zur Mitnahme des Kopfabschnitts 200 durch den Aktuator 202 dienen.

Ferner ist ein Schirm 210 vorhanden, der dazu bestimmt ist, am distalen Endbereich 50 des intraabdominalen Abschnitts 70 befestigt zu sein.

Der Schirm 210 weist einen wenigstens annähernd kreiszylinderförmigen Grundkörper 211 zur Befestigung an dem intraabdominalen Abschnitt 70 und an dem Grundkörper 211 fest angeordnete Schirmsegmente 213 auf.

Der Schirm 210 ist im Anwendungsfall an dem distalen Endbereich 50 des intraabdominalen Abschnitts 70 über eine Gewindeverbindung 212 am distalen Endbereich 50 des intraabdominalen Abschnitts 70 auswechselbar befestigt. In der Ausführungsform der Figuren 1, 2 und 4 weist der intraabdominale Abschnitt 70 ein Aussengewinde 214 und der Grundkörper 211 ein mit diesem Aussengewinde zusammenwirkendes Innengewinde 216, sodass der Schirm 210 auf den intraabdominalen Abschnitt aufgeschraubt werden kann.

In der Ruheposition des Schirms 210 verlaufen der Schirm 210 und somit die Schirmsegmente 213 zusammengefaltet im Wesentlichen parallel zur Längsachse L des Führungselements 20, wie in Fig. 1 abgebildet. Die Schirmsegmente 213 stehen unter einer Vorspannung in Richtung Aufspannposition, um bei Freigabe sich selbsttätig in die Aufspannposition zu bewegen.

In der Aufspannposition des Schirms 210 verlaufen der Schirm 210 und somit die Schirmsegmente 213 im Wesentlichen nach distal in radialer Richtung von der Längsachse des Führungselements 20 weg, wie in Fig. 4 und Fig. 5 sowie Fig. 7 und Fig. 8 abgebildet, um ein trichterförmiges Haltemittel für das zu zerkleinernde Körpergewebe zu bilden.

In axialer Richtung gemessen, steht das distale Ende 220 des in der maximalen Aufspannposition aufgespannten, montierten Schirms 210, bei vollständig eingeführtem Zerkleinerungsinstrument 142, dem freien Ende 172 des Zerkleinerungs- und Förderabschnitts 170 gegenüber vor, wie dies auf Fig. 4 und Fig. 8 zu sehen ist.

Ferner ist eine auf den intraabdominalen Abschnitt 70 aufschiebbare Schiebehülse 222 vorgesehen, welche im montierten Zustand auf den intraabdominalen Abschnitt 70 zwischen einer Freigabeposition und einer Klemmposition hin und her schiebbar ist. Die Schiebhülse 222 ist zylinderförmig und weist einen Innendurchmesser auf, welcher grösser als der Aussendurchmesser des intraabdominalen Abschnitts 70 ist, sodass sie über den intraabdominalen Abschnitt 70 geschoben werden kann.

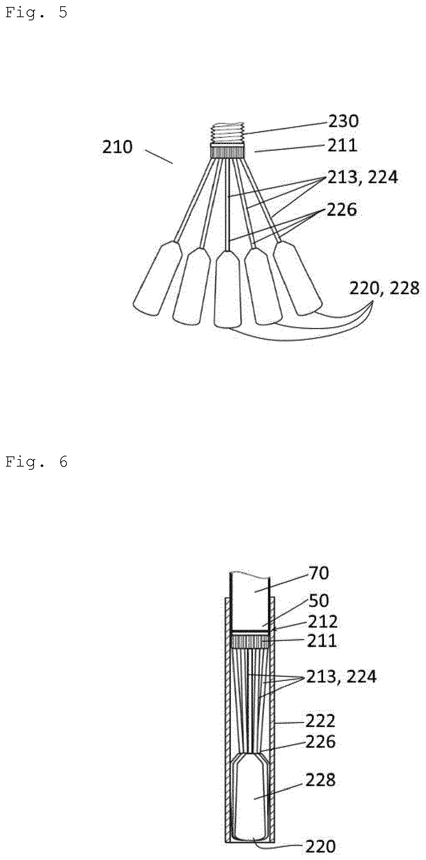

Die Schiebehülse 222 ist in Fig. 1 und Fig. 6 in der Klemmposition dargestellt, in der sie den Schirm 210 umgreift und diesen in seiner Ruheposition hält.

In Fig. 4 ist die Schiebehülse 222 nach ihrer Bewegung nach proximal entlang des intraabdominalen Abschnitts 70 in der Freigabeposition dargestellt, in der sie das Aufspannen des Schirms 210 in seine Aufspannposition ermöglicht.

In der auf die Figuren 1, 2 und 4 dargestellten Ausführungsform weist der Schirm 210 sechs Schirmsegmente 213, welche durch zur Längsachse L konvexe, trapezförmige und nach distal sich verjüngende, vom Grundkörper 211 abstehende Platten ausgebildet sind. Die Schirmsegmente 213 sind in Umfangsrichtung am Grundkörper 211 gleichmässig verteilt und gleich ausgebildet und weisen jeweils ein abgerundetes distales Ende 220 auf.

Bei dem in der Fig. 5 und Fig. 6 dargestellten Schirm 210 sind die Schirmsegmente 213 durch vom Grundkörper 211 abstehende Speichen 224 und an deren freien Enden 226 anschliessende, zungenförmige Platten 228 gebildet. Die Schirmsegmente 213 sind in Umfangsrichtung am Grundkörper 211 gleichmässig verteilt und gleich ausgebildet und die Platten 228 weisen jeweils ein abgerundetes distales Ende 220 auf.

Der Schirm 210 ist im Anwendungsfall, wie in Fig. 6 dargestellt, an dem distalen Endbereich 50 des intraabdominalen Abschnitts 70 über eine Gewindeverbindung 212 am distalen Endbereich 50 des intraabdominalen Abschnitts 70 auswechselbar befestigt. Der Grundkörper 211 weist ein Aussengewinde 230 auf, welches mit einem Innengewinde des intraabdominalen Abschnitts 70 zusammenwirken kann, sodass der Schirm 210 auf den intraabdominalen Abschnitt 70 aufgeschraubt werden kann.

Ferner ist die auf den intraabdominalen Abschnitt 70 aufschiebbare Schiebehülse 222 auf Fig. 6 im montierten Zustand auf den intraabdominalen Abschnitt 70 in ihrer Klemmposition dargestellt.

In Fig. 7 ist ein Schritt der Operation abgebildet, nachdem das zu zerkleinerte Körpergewebe 230 in einen in die Körperhöhle 72 über den Hautschnitt 74 eingeführten Schutzbeutel 232 hineingelegt und die Öffnung 234 des Schutzbeutels 232 aus der Körperhöhle 72 herausgezogen wurde. Die Körperhöhle 72 kann beispielsweise eine Bauchhöhle sein, welche durch eine Bauchdecke 236 veranschaulicht ist.

In diesem Schritt der Operation wurde das den montierten Schirm 210 aufweisende Führungselement 20 in die Körperhöhle 72 über die Öffnung 234 des Schutzbeutels 232 eingeführt und danach der Schirm 210 aufgespannt. Zur Aufspannung des Schirmes 210 wurde die Schiebhülse 222 in ihre Freigabeposition nach proximal entlang des intraabdominalen Abschnitts 70 geschoben. Zur Vereinfachung der Abbildung ist jedoch die Schiebhülse 222 in ihre Freigabeposition auf Fig. 7 nicht dargestellt.

Der aufgespannte Schirm 210 wurde über die Bedienung des Führungselement 20 oberhalb des zu zerkleinerten Körpergewebe 230 positioniert, sodass das zu zerkleinerte Körpergewebe 230 zwischen den Schirmsegmenten 213 und den Schutzbeutel 232 gehalten wird.

Auf Fig. 8 ist ein späterer weiterer Schritt der Operation abgebildet, in welchem das Zerkleinerungsinstrument 142 ins Führungselement 20 eingeführt wurde.

Der durch das Führungselement 20 maskierte Teil des eingeführten Zerkleinerungsinstruments 142 ist gestrichelt dargestellt.

Aus Fig. 8 ist ersichtlich, dass das distale Ende 220 des in der maximalen Aufspannposition aufgespannten, montierten Schirms 210, bei vollständig eingeführtem Zerkleinerungsinstrument 142, in axialer Richtung L gemessen, gegenüber dem freien Ende des Zerkleinerungsinstruments 142, d.h. gegenüber dem freien distalen Ende 172 des Zerkleinerungs- und Förderabschnitts 164, vorsteht. Somit zerkleinert das Zerkleinerungsinstruments 142 das zu zerkleinerte Körpergewebe 230 ohne den Schutzbeutel 232 und das umliegende Gewebe zu verletzen.

Ferner ist aus Fig. 8 ersichtlich, wie der Schutzbeutel 232 von der Aussenseite der Körperhöhle 72 durch Ziehen straff gegen das zu zerkleinernde Körpergewebe 230 und das distale Ende 220 des Schirms 210 gehalten wird, um die Zerkleinerung durch das Zerkleinerungsinstrument 142 zu unterstützen.

Die Darstellung in den Zeichnungen beschränkt sich auf diejenigen Teile der Vorrichtung, die zum Verständnis der betreffenden Ausführungsformen unbedingt notwendig sind. Nicht dargestellt sind beispielsweise die Handhabevorrichtung und der Saugschlauch.

### Liste der Bezugszeichen

Längsachse L
Förderdrehrichtung D
chirurgische Vorrichtung 10
Führungselement 20
Durchgangskanal 30
Abführöffnung 40
distales Ende des Führungselements 50
proximales Ende des Führungselements 60
intraabdominaler Abschnitt 70
Hautschnitt 74
Körperhöhle 72
Abführabschnitt 80
Proximales Ende des intraabdominalen Abschnitts, distales Ende des Abführabschnitts 90
erster Bereich des Abführabschnitts 92
zweiter Bereich des Abführabschnitts 94
dritter Bereich des Abführabschnitts 96
proximales Ende des Abführabschnitts 98
Kammer 100
Anschluss 102
distaler bzw. proximaler Gehäusebereich 110 bzw. 120
Gewindeverbindung 122
Dichtungsabschnitt 130
proximales Ende des Dichtungsabschnitts 132
zylindrischer Bereich des Dichtungsabschnitts 140
Zerkleinerungsinstrument 142
Sitzbereich 150
Anschlag 160
Gegenanschlag 162
distaler Endbereich des Zerkleinerungsinstruments 164
Förderschnecke 166
Zerkleinerungs- und Förderabschnitt 170
distales Ende des Zerkleinerungsinstruments 172
Schaftabschnitt 174
Helix 180
Spalt 182
inneren Wandung des intraabdominalen Abschnitts 184
Teil der Förderschnecke, Bohrabschnitt 186
Führungsabschnitt 190
Dichtungssektion 192
Anschlagssektion 194
Kopfabschnitt 200
Aktuator 202
Nuten 204
Schirm 210
Grundkörper 211
Gewindeverbindung 212
Schirmsegmente 213
Aussengewinde 214
Innengewinde 216
distales Ende des Schirms 220
Schiebehülse 222
Speiche 224
freies Ende der Speiche 226
Platten 228
das zu zerkleinerte Körpergewebe 230
Schutzbeutel 232
Öffnung des Schutzbeutels 234

## Patentansprüche

1. Chirurgische Vorrichtung (10) zum Zerkleinern und Abtransportieren von Körpergewebe, umfassend ein, eine Längsachse (L) der Vorrichtung (10) definierendes Führungselement (20), welches einen, sich wenigstens annähernd rotationssymmetrisch entlang der Längsachse (L) erstreckenden Durchgangskanal (30) und eine mit dem Durchgangskanal (30) strömungsverbundene Abführöffnung (40) zur Abführung vom zerkleinerten Körpergewebe aufweist, wobei der sich distal zur Abführöffnung erstreckender Bereich des Führungselements einen wenigstens annähernd zylindrischen, intraabdominalen Abschnitt (70) aufweist, welcher dazu bestimmt ist, in eine Körperhöhle eingeführt zu werden, und ein in den Durchgangskanal einführbares, schaftförmiges Zerkleinerungsinstrument(142), welches zumindest in seinem distalen Endbereich (164) einen eine Förderschnecke (166) aufweisenden Zerkleinerungs- und Förderabschnitt (170) zum Zerkleinern und Abtransportieren vom Körpergewebe umfasst, im Anwendungsfall den Durchgangskanal (30) durchgreift, und dazu bestimmt ist, von einem Aktuator (202) rotierend angetrieben zu werden,
**dadurch gekennzeichnet, dass** bei vollständig eingeführtem Zerkleinerungsinstrument (142) der Zerkleinerungs- und Förderabschnitt (170) des Zerkleinerungsinstruments (142) über das distale Ende (50) des Führungselements (20) in axialer Richtung vorsteht, um Körpergewebe zu zerkleinern und abzutransportieren, und dass ein Schirm (210) vorhanden ist, welcher dazu bestimmt ist, an einem distalen Endbereich (50) des intraabdominalen Abschnitts (70) befestigt zu sein, wobei der Schirm (210) in seiner Ruheposition zusammengefaltet im Wesentlichen parallel zur Längsachse des Führungselements (20) und in seiner Aufspannposition im Wesentlichen nach distal in radialer Richtung von der Längsachse (L) des Führungselements (20) weg verläuft, um ein trichterförmiges Haltemittel für das zu zerkleinernde Körpergewebe zu bilden.

2. Vorrichtung (10) nach Anspruch 1, **dadurch gekennzeichnet, dass** der Schirm (210) an dem distalen Endbereich (50) des intraabdominalen Abschnitts (70) auswechselbar befestigbar ist.

3. Vorrichtung (10) nach Anspruch 2, **dadurch gekennzeichnet, dass** der Schirm (210) über eine Gewindeverbindung (212) am distalen Endbereich (50) des intraabdominalen Abschnitts (70) lösbar befestigbar ist.

4. Vorrichtung (10) nach Anspruch 2, **dadurch gekennzeichnet, dass** der Schirm (210) über einen Bajonettverschluss befestigbar ist.

5. Vorrichtung (10) nach einem der Ansprüche 1 bis 4, **dadurch gekennzeichnet, dass**, in axialer Richtung gemessen, das distale Ende des in der maximalen Aufspannposition aufgespannten, montierten Schirms (210), bei vollständig eingeführtem Zerkleinerungsinstrument (142), bei dem freien Ende (172) des Zerkleinerungs- und Förderabschnitts (170) liegt, vorzugsweise gegenüber diesem vorsteht.

6. Vorrichtung (10) nach einem der Ansprüche 1 bis 5, **gekennzeichnet durch** eine auf den intraabdominalen Abschnitt (70) aufschiebbare Schiebehülse (222), welche im montierten Zustand auf den intraabdominalen Abschnitt (70) zwischen einer Freigabeposition und einer Klemmposition hin und her schiebbar ist, wobei die Schiebehülse (222) in ihrer Klemmposition den Schirm (210) umgreift und in seiner Ruheposition hält, und die Schiebehülse (222) dazu bestimmt ist, nach proximal entlang des intraabdominalen Abschnitts (70) in ihre Freigabeposition bewegt zu werden, um das Aufspannen des Schirms (210) in seine Aufspannposition freizugeben.

7. Vorrichtung (10) nach einem der Ansprüche 1 bis 6, **dadurch gekennzeichnet, dass** das Führungselement (20) einen proximal zur Abführöffnung (40) angeordneten Dichtungsabschnitt (130) und das Zerkleinerungsinstrument (142) eine, Dichtungssektion (192) aufweisen, wobei der Dichtungsabschnitt (130) und die Dichtungssektion (192) derart ausgebildet sind, dass sie sich mindestens teilweise überlappen, wenn das distale Ende (172) des Zerkleinerungs- und Förderabschnitts (170) über das distale Ende (50) des Führungselements (20) in axialer Richtung vorsteht, um eine wenigstens annähernd vollständig luftdichte Abdichtung des Führungselements (20) proximal zur Abführöffnung (40) zu bilden.

8. Vorrichtung (10) nach einem der Ansprüche 1 bis 7, **dadurch gekennzeichnet, dass** der Schirm (210) einen Grundkörper (211) zur Befestigung an dem intraabdominalen Abschnitt (70) und an den Grundkörper (211) fest angeordnete Schirmsegmente (213) aufweist, wobei Schirmsegmente (213) in der Ruheposition im Wesentlichen parallel zur Längsachse des Führungselements (20) verlaufen und unter einer Vorspannung in Richtung Aufspannposition stehen, um bei Freigabe sich selbsttätig in die Aufspannposition zu bewegen, und in der Aufspannposition im Wesentlichen nach distal in radialer Richtung von der Längsachse (L) des Führungselements (20) weg verlaufen.

9. Vorrichtung (10) nach einem der Ansprüche 1 bis 7, **dadurch gekennzeichnet, dass** der Schirm (210) einen Grundkörper (211) zur Befestigung an dem intraabdominalen Abschnitt (70) und an den Grundkörper (211) schwenkbar zwischen der Ruheposition und der Aufspannposition am Grundkörper (211) gelagerte Schirmsegmente (213) aufweist, und ein mit den Schirmsegmenten (213) über ein Verbindungselement verbundenes Stellglied vorhanden ist, wobei der Wechsel zwischen der Ruheposition und der Aufspannposition durch die Betätigung des Stellglieds erfolgt.

10. Vorrichtung (10) nach einem der Ansprüche 8 oder 9, **dadurch gekennzeichnet, dass** die Schirmsegmente (213) durch zur Längsachse (L) konvexe, trapezförmige und nach distal sich verjüngende Platten ausgebildet sind, welche vom Grundkörper (211) abstehen.

11. Vorrichtung (10) nach einem der Ansprüche 8 bis 10, **dadurch gekennzeichnet, dass** die Schirmsegmente (213) durch vom Grundkörper (211) abstehende Speichen (224) und an deren freien Enden (226) anschliessende, zungenförmige Platten (228) gebildet sind.

12. Vorrichtung (10) nach einem der Ansprüche 8 bis 11, **dadurch gekennzeichnet, dass** der Schirm (210) 3 bis 12, bevorzugt 4 bis 6, besonders bevorzugt 4 Schirmsegmente (213)aufweist.

13. Einwegschirm (210) für eine Vorrichtung (10) nach einem der Ansprüche 1 bis 12, wobei der Einwegschirm (210) austauchbar ist.
